# FASCICULE DE BREVET EUROPEEN

(11) **EP 2 398 423 B1**
(45) Date de publication et mention de la délivrance du brevet: **31.10.2012**
(21) Numéro de dépôt: 10708348.7
(22) Date de dépôt: 15.02.2010
(51) Int. Cl.: A61F 2/44

(54) **PROTHÈSE DE DISQUE INTERVERTÉBRAL**
BANDSCHEIBENPROTHESE
INTERVERTEBRAL DISC PROSTHESIS

(30) Priorité: 18.02.2009 FR 0951047
(43) Date de publication de la demande: 28.12.2011
(73) Titulaire: Fournitures Hospitalieres Industrie, 29000 Quimper (FR)
(72) Inventeur: LAZENNEC, Jean-Yves, F-94240 L'Hay les Roses (FR); SAILLANT, Gérard, F-78170 La Celle St Cloud (FR); LAVILLE, Claude, F-75016 Paris (FR); PASCAL-MOUSSELLARD, Hugues, F-75015 Paris (FR); RAKOVER, Jean-Patrick, F-72700 Rouillon (FR); RICART, Olivier, L-3489 Dudelange (LU); AARON, Alain, F-95470 Saint Witz (FR)
(74) Mandataire: Maureau, Philippe
(86) Numéro de dépôt international: PCT/FR2010/050251
(87) Numéro de publication internationale: WO 2010/094881

(56) Documents cités:
- EP-A- 1 214 918
- EP-A- 2 018 832
- WO-A1-2008/010240
- FR-A- 2 761 878
- FR-A- 2 787 021
- FR-A- 2 863 868
- US-A1- 2005 143 747
- US-A1- 2006 287 728
- US-A1- 2008 195 206

## Description

La présente invention concerne une prothèse destinée à remplacer un disque intervertébral endommagé de la colonne vertébrale, et plus particulièrement un disque intervertébral cervical endommagé de la colonne vertébrale.

La colonne vertébrale est constituée par un ensemble de vertèbres superposées reliées les unes aux autres par des disques fibro-cartilagineux, appelés disques intervertébraux. Ces disques intervertébraux ont un rôle fondamental dans la statique et la dynamique de la colonne vertébrale : ils assurent la mobilité des vertèbres entre elles.

Ces disques intervertébraux sont souvent l'objet de désordres relatifs à un tassement de vertèbres, une hernie discale, un déplacement de vertèbres ou encore une dégénérescence arthrosique intervertébrale. Ces désordres sont, bien souvent, la cause de douleurs ou de gènes fonctionnelles rebelles aux traitements médicaux ; dans certains cas, ils peuvent même être invalidants.

Le procédé utilisé pour soulager les patients atteints de ces désordres consiste, généralement, en une intervention chirurgicale. Plusieurs techniques d'intervention chirurgicale sont actuellement connues.

Une première technique d'intervention chirurgicale consiste en une arthrodèse intervertébrale par laquelle les deux vertèbres adjacentes au disque endommagé sont fusionnées. Ce procédé est, en effet, un remède définitif qui bloque l'évolution arthrosique, c'est-à-dire la dégradation, dans le disque. Ce procédé a pour principal inconvénient de supprimer toute mobilité entre les deux vertèbres adjacentes au disque endommagé. Cette suppression de mobilité des vertèbres adjacentes au disque endommagé a pour conséquence de concentrer les contraintes mécaniques sur les disques intervertébraux adjacents, ce qui peut entraîner un risque de détérioration de leur surface articulaire.

Une deuxième technique consiste en une chirurgie arthroplastique intervertébrale de remplacement du disque endommagé par une prothèse de disque intervertébral.

Une telle prothèse est décrite, par exemple, dans le document FR-A-2 124 815. Cette prothèse consiste, plus précisément, en un élément affectant la forme d'un disque intervertébral et réalisé dans un matériau élastomère du type silicone. Elle a pour inconvénient de ne pouvoir assurer qu'une mobilité limitée des vertèbres adjacentes, son aptitude à se déformer étant relativement limitée. En outre, cette prothèse n'interdit pas l'expansion radiale du matériau élastomère lors des contraintes exercées sur la prothèse, ce qui se traduit par un risque d'herniation vers le canal médullaire.

Le document FR 2 709 949 concerne une prothèse de disque intervertébral comprenant deux demi-enveloppes en forme de coupelles, fixées chacune sur une des deux vertèbres adjacentes au disque vertébral à remplacer, entre lesquelles est disposé un coussin de compression entouré par une ceinture.

Une telle prothèse présente l'inconvénient d'un manque de fiabilité en raison des risques de désolidarisation du coussin et des coupelles sous l'effet des forces de cisaillement qui s'exercent entre le coussin et les coupelles, notamment lorsque la prothèse est en position inclinée par rapport à l'horizontale, en conditions d'utilisation.

Le frottement du coussin sur la ceinture peut générer des particules d'usure susceptibles d'entraîner des désordres en particulier neurologiques. En outre, la désolidarisation du coussin des coupelles peut entraîner également un risque de déplacement de ce coussin, qui peut entrer en conflit avec des parties vitales, notamment neurologiques, vasculaires ou aéro-digestives.

Le document FR 2 863 868 concerne une prothèse de disque intervertébral comportant deux demi-enveloppes rigides en forme de coupelles, fixées chacune sur une des deux vertèbres adjacentes au disque intervertébral à remplacer, les deux demi-enveloppes enserrant un coussin de compression. L'une des deux demi-enveloppes comporte dans sa zone centrale une cheminée tournée vers l'autre demi-enveloppe. L'autre demi-enveloppe comporte dans sa zone centrale un plot de section inférieure à celle de la cheminée, tourné vers la première demi-enveloppe et engagé dans la cheminée de celle-ci, le coussin de compression étant disposé entre les deux demi-enveloppes, y compris dans le volume compris entre la cheminée et le plot.

Cette structure permet à la prothèse de parfaitement résister aux contraintes de cisaillement, puisque celles-ci ne sont plus absorbées, comme tel est le cas habituellement par les seules interfaces entre le coussin de compression et les coupelles, avec risque de désolidarisation subséquent, mais aussi par compression entre le plot central et la paroi de la cheminée. En outre il faut noter qu'en cas de rupture à l'interface entre le coussin et l'une des demi-enveloppes, la sécurité est assurée dans la mesure où le couple plot-cheminée va limiter le déplacement relatif des deux demi-enveloppes rigides.

Bien que ce type de prothèse de disque intervertébral présente une parfaite sécurité d'utilisation, il a été constaté que ce type de prothèse de disque intervertébral présente une certaine rigidité qui ne correspond pas aux caractéristiques d'un disque intervertébral naturel.

La présente invention vise à remédier à ces inconvénients.

Le problème technique à la base de l'invention consiste donc à fournir une prothèse de disque intervertébral qui soit d'une réalisation simple, d'une structure compacte, et qui présente une parfaite sécurité d'utilisation, tout en comportant des caractéristiques proches de celles d'un disque intervertébral naturel.

A cet effet, l'invention concerne une prothèse de disque intervertébral selon la revendication 1.

La prothèse de disque intervertébral selon l'invention présente les avantages de celle décrite dans le document FR 2 863 868, tout en présentant des caractéristiques plus proches de celles d'un disque intervertébral naturel du fait de la structure du coussin de compression qui assure une diminution de la rigidité de la prothèse.

De préférence, les première, deuxième, troisième et quatrième branches du coussin de compression s'étendent sensiblement perpendiculairement à l'axe de la portion centrale.

De façon préférentielle, la longueur des troisième et quatrième branches est inférieure à celle des première et deuxième branches. Avantageusement, la longueur des troisième et quatrième branches est inférieure d'environ 20% à celle des première et deuxième branches. Ces dispositions permettent de créer une raideur supérieure en flexion latérale que celle en flexion / extension.

Selon une variante de réalisation de la prothèse, la largeur de chaque branche décroît de manière continue en direction de l'extrémité libre de ladite branche.

Avantageusement, le coussin de compression est réalisé en une seule pièce.

Préférentiellement, chaque demi-enveloppe comporte, sur sa face intérieure, c'est-à-dire sur sa face tournée vers l'autre demi-enveloppe, des moyens favorisant l'accrochage du coussin de compression.

Selon une variante de réalisation de la prothèse, chaque demi-enveloppe comporte, sur sa face intérieure, deux rainures s'étendant sensiblement perpendiculairement l'une par rapport à l'autre et dont le point d'intersection est situé sensiblement dans la zone centrale de ladite demi-enveloppe, les deux rainures étant agencées pour permettre l'accrochage des branches du coussin de compression.

Préférentiellement, les deux rainures ménagées sur chaque demi-enveloppe présentent une forme complémentaire de celle des branches du coussin de compression.

De façon avantageuse, les rainures ménagées sur chaque demi-enveloppe présentent une section en queue d'aronde.

De préférence, le coussin de compression est réalisé en un matériau présentant une dureté shore D comprise entre 60 et 100 et de préférence de 80.

Selon une variante de réalisation de la prothèse, au moins l'une des demi-enveloppes comporte, sur sa face extérieure, c'est-à-dire sa face opposée au coussin de compression, des pointes de fixation destinées à favoriser sa fixation sur une vertèbre.

Avantageusement, la surface externe d'au moins l'une des demi-enveloppes est recouverte d'un premier revêtement rugueux comportant du titane pur, tel que le titane T40, et d'un second revêtement de phosphate de calcium, tel que l'hydroxyapatite, disposé sur le premier revêtement.

De façon avantageuse, l'extrémité libre de chacune des première et seconde branches est délimitée extérieurement par au moins une surface concave s'étendant entre les faces intérieures des première et seconde demi-enveloppes, et de préférence s'étendant de la face intérieure de la première demi-enveloppe à la face intérieure de la seconde demi-enveloppe.

Préférentiellement, au moins l'une des branches du coussin de compression, et de préférence au moins l'une des troisième et quatrième branches, comporte au moins une portion en saillie s'étendant le long de la face intérieure de l'une des demi-enveloppes et dont la hauteur est inférieure à la distance séparant les deux demi-enveloppes. Selon un mode de réalisation de l'invention, chacune des troisième et quatrième branches comporte au moins une portion en saillie. La présence de telles portions en saillie permet d'éviter un conflit métal sur métal entre les deux demi-enveloppes en position extrême de flexion-extension.

Les portions en saillie peuvent par exemple s'étendre le long de la face intérieure d'une même demi-enveloppe. Selon une variante de réalisation, l'une des portions en saillie s'étend le long de la face intérieure de l'une des demi-enveloppes, tandis que l'autre portion en saillie s'étend le long de la face intérieure de l'autre demi-enveloppe.

De préférence, chaque demi-enveloppe comporte, sur sa face intérieure, deux empreintes débouchant dans la face antérieure de la demi-enveloppe correspondante, les empreintes de la première demi-enveloppe étant situées en regard des empreintes de la seconde demi-enveloppe de manière à former deux cavités convergeant en direction de la face antérieure de la prothèse.

De toute façon l'invention sera bien comprise à l'aide de la description qui suit en référence au dessin schématique annexé représentant, à titre d'exemples non limitatifs, plusieurs formes d'exécution de cette prothèse de disque intervertébral.
Figure 1 et 2 sont des vues explosées en perspective d'une prothèse de disque intervertébral selon une première variante de réalisation de l'invention.
Figure 3 est une vue de côté de la prothèse de figure 1.
Figure 4 est une vue explosée en perspective d'une prothèse de disque intervertébral selon une deuxième variante de réalisation de l'invention.
Figure 5 est une vue de côté de la prothèse de figure 4.
Figure 6 est une vue de dessus d'une prothèse de disque intervertébral selon une troisième variante de réalisation de l'invention.
Figure 7 est une vue partielle en coupe de la demi-enveloppe supérieure de la prothèse de figure 5.
Figure 8 est une vue en coupe d'une prothèse de disque intervertébral selon une quatrième variante de réalisation de l'invention.
Figure 9 est une vue de côté d'une prothèse de disque intervertébral selon une cinquième variante de réalisation de l'invention.
Figure 10 est une vue en perspective de la demi-enveloppe inférieure de la prothèse de figure 9.
Figure 11 est une vue en perspective de la demi-enveloppe supérieure de la prothèse de figure 9.
Figure 12 est une vue en perspective de la demi-enveloppe supérieure et du coussin de compression de la prothèse de figure 9.
Figure 13 est une vue en coupe dans le plan médio latéral de la prothèse de figure 9.
Figure 14 est une vue en perspective des deux demi-enveloppes de la prothèse de figure 9 en position de fonctionnement.

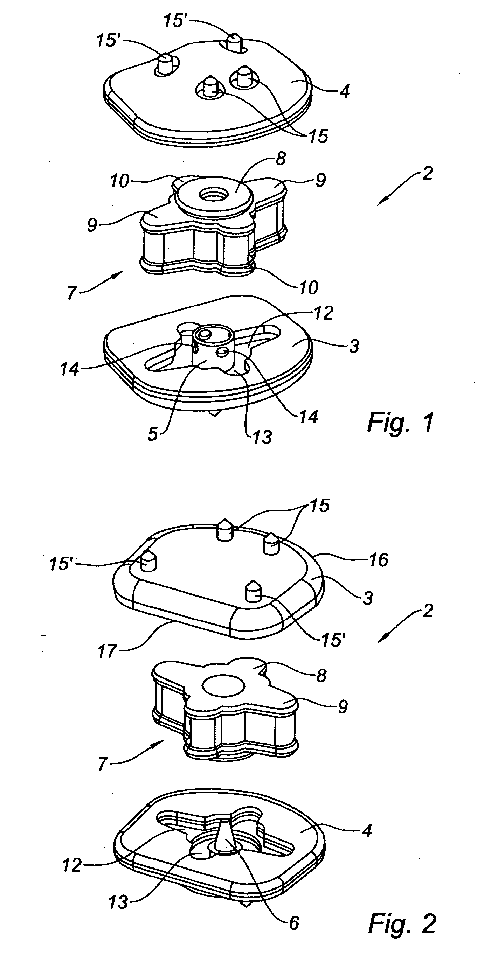

Les figures 1 à 3 représentent une prothèse de disque intervertébral cervical 2 destinée à remplacer un disque intervertébral cervical endommagé.

Cette prothèse 2 comporte une demi-enveloppe inférieure 3 et une demi-enveloppe supérieure 4 en forme de plateaux. Les deux demi-enveloppes 3, 4 sont réalisées de préférence en alliage à base de titane et sont destinées chacune à être fixées sur une des deux vertèbres cervicales adjacentes au disque intervertébral à remplacer. Les deux demi-enveloppes 3, 4 sont de forme générale rectangulaire.

Comme montré sur la figure 1, la demi-enveloppe inférieure 3 comporte, dans sa zone centrale, une pièce tubulaire 5 de section circulaire tournée vers la demi-enveloppe supérieure 4. La pièce tubulaire 5 présente de préférence une longueur de l'ordre de 2 à 3 mm, une épaisseur comprise entre 0,5 et 1 mm, et un diamètre intérieur compris entre 3 et 5 mm.

Comme montré sur la figure 2, la demi-enveloppe supérieure 4 comporte, dans sa zone centrale, un plot tronconique 6 de section inférieure à celle de la pièce tubulaire 5, tourné vers la demi-enveloppe inférieure 3 et engagé dans la pièce tubulaire 5. Le plot tronconique 6 présente de préférence une longueur de l'ordre de 2 à 3 mm, et un diamètre tel qu'il existe un volume annulaire entre le plot tronconique 6 et la pièce tubulaire 5 présentant une largeur comprise entre 0,8 et 1,2 mm.

Il doit être noté que la somme des longueurs de la pièce tubulaire 5 et du plot 6 est supérieure à la distance entre les deux demi-enveloppes 3, 4.

La prothèse 2 comporte en outre un coussin de compression 7 disposé entre les deux demi-enveloppes 3, 4, y compris dans le volume compris entre la pièce tubulaire 5 et le plot 6. Le coussin de compression 7 est réalisé avantageusement en un matériau compressible, de préférence de type polycarbonate uréthane. De façon préférentielle, le coussin de compression 7 est réalisé en un matériau présentant une dureté shore D d'environ 80.

Comme montré sur les figures 1 et 2, le coussin de compression 7 présente sensiblement une forme de croix dont le centre est situé dans les zones centrales des demi-enveloppes 3, 4. Le coussin de compression comporte plus particulièrement une portion centrale 8 s'étendant entre les zones centrales des première et seconde demi-enveloppes selon un axe sensiblement perpendiculaire aux première et seconde demi-enveloppes, et quatre branches s'étendant perpendiculairement à partir de la portion centrale 8 et régulièrement espacées les unes des autres.

Le coussin de compression 7 comporte une première et une deuxième branches 9 s'étendant sensiblement dans le plan médio latéral de la prothèse, et une troisième et une quatrième branches 10 s'étendant perpendiculairement aux première et deuxième branches 9, c'est-à-dire dans le plan antéro postérieur.

La longueur des troisième et quatrième branches est de préférence inférieure d'environ 20% à celle des première et deuxième branches.

Chaque demi-enveloppe 3, 4 comporte, sur sa face intérieure, c'est-à-dire sur sa face tournée vers l'autre demi-enveloppe, deux rainures 12, 13 s'étendant sensiblement perpendiculairement l'une par rapport à l'autre et dont le point d'intersection est situé sensiblement dans la zone centrale de ladite demi-enveloppe. Les deux rainures 12, 13 sont agencées pour permettre l'accrochage des branches 9, 10 du coussin de compression 7. Elles présentent de ce fait une forme complémentaire de celle des branches du coussin de compression 7.

De préférence, les rainures 12, 13 ménagées sur chaque demi-enveloppe présentent une section en queue d'aronde. Les parois délimitant latéralement chaque rainure en queue d'aronde forment avantageusement un angle d'environ 60° par rapport au fond de ladite rainure.

Plus particulièrement, le point d'intersection des rainures 12, 13 ménagées sur la demi-enveloppe inférieure 3 s'étend selon l'axe de la pièce tubulaire 5, et le point d'intersection des rainures 12, 13 ménagées sur la demi-enveloppe supérieure 4 s'étend selon l'axe du plot 6.

La rainure 12 ménagée sur chaque demi-enveloppe s'étend sensiblement selon l'axe médio latéral de ladite demi-enveloppe, tandis que la rainure 13 ménagée sur chaque demi-enveloppe s'étend sensiblement perpendiculairement à la rainure 12 correspondante.

Afin de favoriser l'accrochage du coussin de compression sur la demi-enveloppe inférieure 3, la pièce tubulaire 5 comporte, comme cela est montré sur la figure 1, une pluralité de lumières 14.

La demi-enveloppe inférieure 3 comporte, sur sa face extérieure, c'est-à-dire sa face opposée au coussin de compression 7, quatre pointes de fixation 15, 15' destinées à favoriser sa fixation sur une vertèbre. Les pointes de fixation 15, 15' s'étendent perpendiculairement à la face extérieure de la demi-enveloppe inférieure 3. Les pointes de fixation 15, 1 5' présentent avantageusement un diamètre compris entre 1 et 1,5 mm et de préférence une longueur comprise entre 2 et 5 mm. Les quatre pointes de fixation 15, 15' sont de préférence conique.

Il convient de noter que parmi ces quatre pointes de fixation, deux pointes de fixation 15 sont disposées selon une première ligne située à proximité de la face antérieure 16 de la demi-enveloppe inférieure 3 et deux pointes de fixation 15' sont disposées selon une seconde ligne située à proximité de la face postérieure 17 de la demi-enveloppe inférieure 3. Les première et seconde lignes sont de préférence parallèles entre elles et parallèles à l'axe médio latéral de la demi-enveloppe inférieure 3.

Avantageusement, les deux pointes de fixation 15 disposées selon la première ligne présente un entraxe inférieur à l'entraxe des deux pointes de fixation 15' disposées selon la seconde ligne.

Comme montré sur la figure 1, la demi-enveloppe supérieure 4 comporte également, sur sa face extérieure, quatre pointes de fixation 15, 15' destinées à favoriser sa fixation sur une vertèbre. Ces quatre pointes de fixation 15, 15' présentent de préférence des dimensions identiques à celles des pointes de fixation ménagées sur la demi-enveloppe inférieure 3. Avantageusement, les quatre pointes de fixation 15, 15' ménagées sur la demi-enveloppe supérieure 4 sont disposées de la même manière que celles ménagées sur la demi-enveloppe inférieure 3.

' De préférence, la face antérieure 16 de la demi-enveloppe inférieure 3 présente un rayon de courbure compris entre 25 et 35 mm, tandis que sa face postérieure 17 est sensiblement plane. Les bords internes et externes de la demi-enveloppe inférieure 3 ont avantageusement des rayons de courbure d'environ 5 mm correspondant à la géométrie moyenne des uncus.

La demi-enveloppe supérieure 4 présente préférentiellement un contour sensiblement identique à celui de la demi-enveloppe inférieure 3 mais réduit de 0,5 mm selon ses dimensions médio latérales afin d'éviter un appui de la demi-enveloppe supérieure sur les uncus osseux.

Comme montré plus particulièrement sur la figure 1, la face supérieure de la demi-enveloppe supérieure 4 présente un profil bombé vers l'extérieur complémentaire de la surface d'articulation de la vertèbre supérieure adjacente au disque à remplacer.

La surface externe des deux demi-enveloppes 3, 4 est recouverte d'un premier revêtement rugueux comportant du titane pur, tel que le titane T40, et d'un second revêtement de phosphate de calcium, tel que l'hydroxyapatite, disposé sur le premier revêtement. Le premier revêtement présente avantageusement une épaisseur comprise entre environ 20 et 40 microns, et de préférence une rugosité de surface de l'ordre de 30 microns.

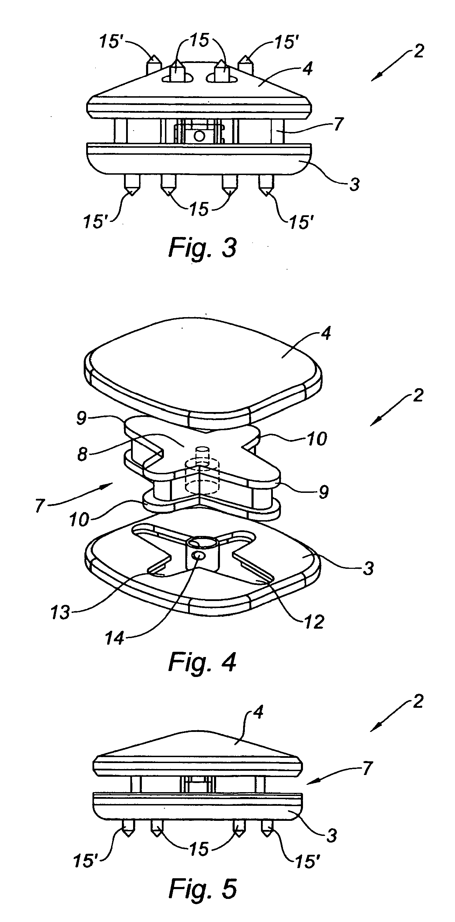

Selon une variante de réalisation de la prothèse montrée sur les figures 4 et 5, la demi-enveloppe supérieure 4 ne comporte pas, sur sa face extérieure, c'est-à-dire sa face opposée au coussin de compression 7, de pointes de fixation 15, 15'.

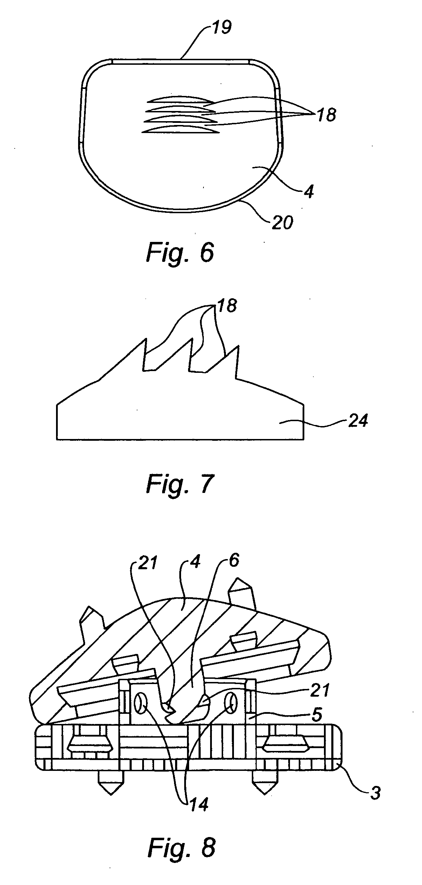

Selon une variante de réalisation de la prothèse montrée sur la figure 6, la demi-enveloppe supérieure 4 comporte, sur sa face extérieure, c'est-à-dire sa face opposée au coussin de compression 7, des nervures d'ancrage 18 s'étendant parallèlement à l'axe médio latéral de la demi-enveloppe supérieure 4. Le nombre de nervures d'ancrage 18 est de préférence compris entre 2 et 5, et avantageusement 3. De façon avantageuse, les nervures d'ancrage 18 s'étendent dans la zone centrale de la face supérieure de la demi-enveloppe supérieure 4. La hauteur de chaque nervure d'ancrage 18 est avantageusement de l'ordre de 0,5 mm.

Comme montré sur la figure 7, les nervures d'ancrage 18 présentent de préférence une section triangulaire dont la pointe s'étend du bas vers le haut et de la face postérieure 19 vers la face antérieure 20 de la demi-enveloppe supérieure 4.

Selon une variante de réalisation de la prothèse montrée sur la figure 8, le plot 6 est cylindrique et présente des évidements 21 agencés de manière à favoriser l'accrochage du coussin de compression 7 sur la demi-enveloppe supérieure 4.

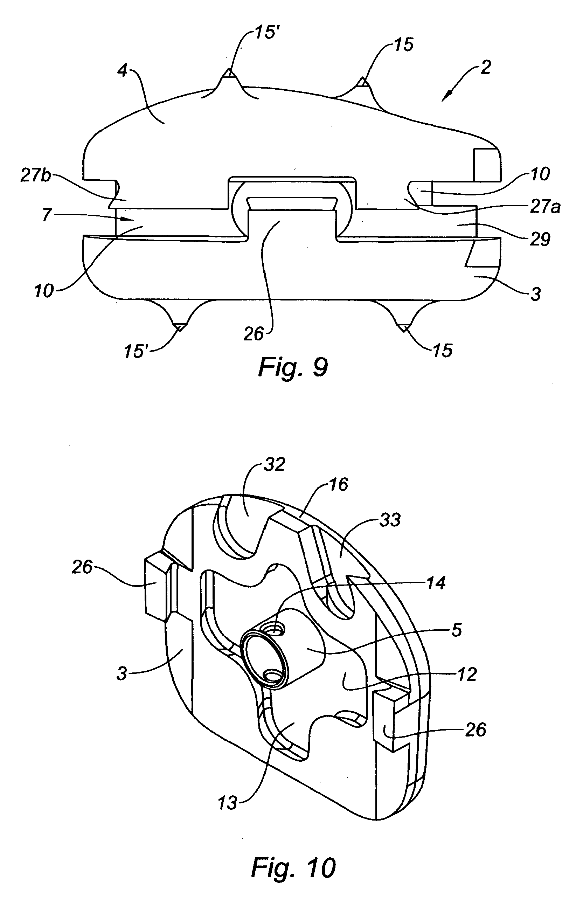

Selon une variante de réalisation de la prothèse montrée sur les figures 9 à 14, l'extrémité libre de chacune des première et seconde branches 9 est délimitée extérieurement par une surface concave 25 s'étendant de la face intérieure de la demi-enveloppe supérieure 4 à la face intérieure de la demi-enveloppe inférieure 3.

Selon cette variante de réalisation, la demi-enveloppe inférieure 3 comporte, sur sa face intérieure et à proximité de chaque extrémité de la rainure 12 recevant les première et seconde branches 9 du coussin de compression 7, un doigt de préhension 26, tandis que la demi-enveloppe supérieure 4 comporte, sur sa face intérieure et à proximité de chaque extrémité de la rainure 12 recevant les première et seconde branches 9 du coussin de compression 7, deux doigts de préhension 27a, 27b.

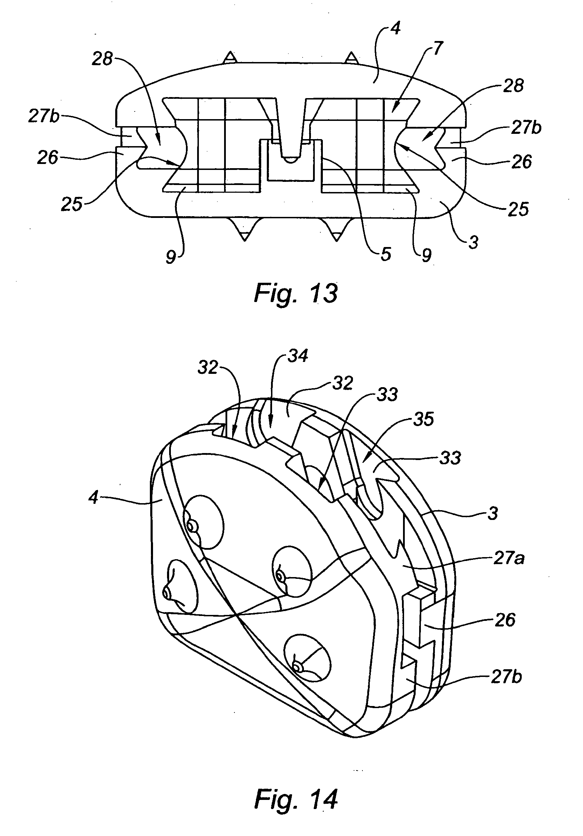

Comme montré sur les figures 9 et 13, les doigts de préhension 26, 27a, 27b situés à proximité d'une même branche 9 du coussin de compression 7 sont décalés angulairement par rapport à la portion centrale 8 du coussin de compression 7 et délimitent avec ladite branche un espace 28 destiné au passage d'un bras de préhension d'un ancillaire. Chaque espace 28 est délimité par trois cotés métalliques, donc indéformables, et un coté du coussin de compression, donc déformable. Chaque bras de l'ancillaire en appui sur les faces inclinées des doigts de préhension et frottant légèrement sur la concavité du coussin de compression assure une solidarisation des demi-enveloppes supérieure et inférieure évitant leur mobilisation lors de l'impaction de la prothèse et évitant la transmission des contraintes d'impaction au coussin de compression. Avantageusement, les dimensions de chaque bras de l'ancillaire sont définies pour assurer une légère compression du coussin de compression, donc une diminution de 0,1 à 0,3 mm de la hauteur totale de la prothèse, facilitant ainsi l'implantation et assurant une meilleure efficacité des pointes de fixation.

Il doit être noté que chaque doigt de préhension 26, 27a, 27b présente une paroi intérieure inclinée par rapport à la face intérieure de la demi-enveloppe correspondante, ladite paroi inclinée formant un coin.

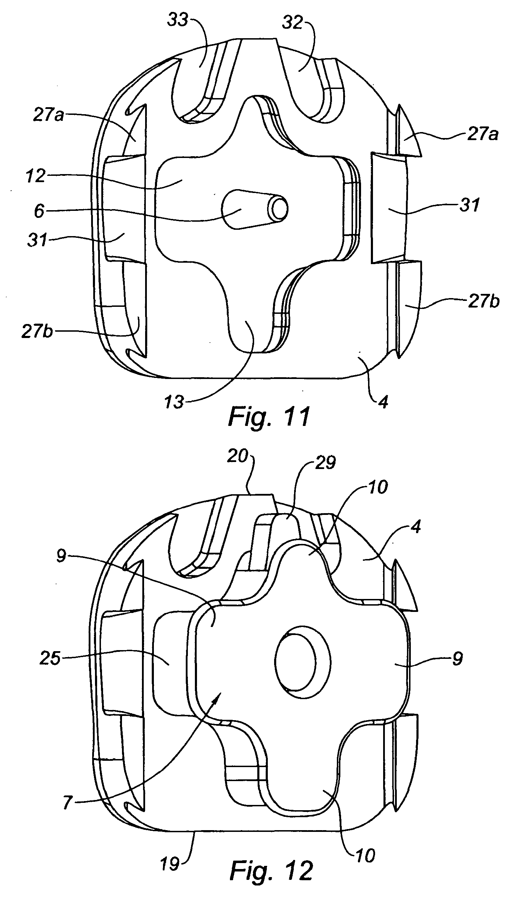

Comme montré plus particulièrement sur les figures 9 et 12, la branche 10 du coussin de compression 7 s'étendant en direction de la face antérieure 16 de la prothèse comporte une portion en saillie 29 s'étendant le long de la face intérieure de la demi-enveloppe supérieure 4. La portion en saillie 29 présente une épaisseur inférieure à la distance séparant les deux demi-enveloppes 3, 4. Selon une variante de réalisation non représentée sur les figures, la branche 10 du coussin de compression 7 s'étendant en direction de la face postérieure 17 de la prothèse pourrait également comporter une portion en saillie s'étendant le long de la face intérieure de l'une des demi-enveloppes, et par exemple de la demi-enveloppe inférieure 3.

Comme montré sur la figure 11, la demi-enveloppe supérieure 4 comprend, sur sa face intérieure et à proximité de chaque extrémité de la rainure 12 recevant les première et seconde branches 9 du coussin de compression 7, un plan incliné 31 disposé entre les doigts de préhension 27a, 27b correspondant. Ces deux plans inclinés 31 permettent d'éviter tout risque de contact entre la demi-enveloppe supérieure 4 et les doigts de préhension 26 de la demi-enveloppe inférieure 3 lors des flexions latérales.

Chaque demi-enveloppe 3, 4 comporte, sur sa face intérieure, deux empreintes 32, 33 débouchant dans la face antérieure 16, 20 de la demi-enveloppe correspondante. Les empreintes 32, 33 de la demi-enveloppe supérieure 4 sont agencées pour être situées en regard des empreintes 32, 33 de la demi-enveloppe inférieure 3 de manière à former deux cavités 34, 35 convergeant en direction de la face antérieure de la prothèse. La présence de ces deux cavités permet l'accrochage d'un extracteur.

Il doit être noté que le procédé de fabrication de la prothèse 2 comprend notamment une étape consistant à traiter par microbillage les surfaces internes des demi-enveloppes, et une étape consistant à traiter par oxydation anodique en milieu basique les surfaces internes des demi-enveloppes après réalisation du traitement de surface par microbillage de celles-ci. De préférence, le traitement de surface par microbillage des surfaces internes des demi-enveloppes est réalisé à l'aide de billes de corindon présentant un diamètre compris entre 300 à 500 microns.

Ces deux étapes successives permettent d'optimiser la liaison entre les demi-enveloppes et le coussin de compression.

Comme il va de soi, l'invention ne se limite pas à la seule forme d'exécution de cette prothèse de disque intervertébral, décrite ci-dessus à titre d'exemple, elle en embrasse au contraire toutes les variantes de réalisation.

## Revendications

1. Prothèse de disque intervertébral (2) comportant des première et seconde demi-enveloppes (3, 4) rigides en forme de coupelles ou de plateaux, destinées chacune à être fixées sur une des deux vertèbres adjacentes au disque intervertébral à remplacer, la première demi-enveloppe (3) comportant dans sa zone centrale une pièce tubulaire (5) tournée vers la seconde demi-enveloppe, la seconde demi-enveloppe (4) comportant dans sa zone centrale un plot (6) de section inférieure à celle de la pièce tubulaire, tourné vers la première demi-enveloppe et engagé dans la pièce tubulaire, la prothèse de disque intervertébral comportant en outre un coussin de compression (7) disposé entre les deux demi-enveloppes, y compris dans le volume compris entre la pièce tubulaire et le plot, le coussin de compression étant solidaire des première et seconde demi-enveloppes, **caractérisée en ce que** le coussin de compression (7) comporte une portion centrale (8) s'étendant entre les zones centrales des première et seconde demi-enveloppes (3, 4) selon un axe sensiblement perpendiculaire aux première et seconde demi-enveloppes, une première et une deuxième branches (9) s'étendant transversalement à partir de la portion centrale (8) et sensiblement dans le plan médio latéral de la prothèse, et une troisième et une quatrième branches (10) s'étendant transversalement à partir de la portion centrale (8) et sensiblement dans le plan antéro postérieur de la prothèse, et **en ce que** chaque demi-enveloppe (3, 4) comporte, sur sa face tournée vers l'autre demi-enveloppe et à proximité de l'extrémité libre de chacune des première et seconde branches (9) du coussin de compression, au moins un doigt de préhension (26, 27a, 27b), les doigts de préhension situés à proximité d'une même branche du coussin de compression (7) étant décalés angulairement par rapport à la portion centrale (8) du coussin de compression et délimitant avec ladite branche un espace (28) destiné au passage d'un bras de préhension d'un ancillaire.

2. Prothèse selon la revendication 1, **caractérisée en ce que** les première, deuxième, troisième et quatrième branches (9, 10) du coussin de compression (7) s'étendent sensiblement perpendiculairement à l'axe de la portion centrale (8).

3. Prothèse selon la revendication 1 ou 2, **caractérisée en ce que** la longueur des troisième et quatrième branches (10) est inférieure à celle des première et deuxième branches (9), et de préférence inférieure d'environ 20% à celle des première et deuxième branches (9).

4. Prothèse selon l'une des revendications 1 à 3, **caractérisée en ce que** la largeur de chaque branche (9, 10) décroît de manière continue en direction de l'extrémité libre de ladite branche.

5. Prothèse selon l'une des revendications 1 à 4, **caractérisée en ce que** le coussin de compression est réalisé en une seule pièce.

6. Prothèse selon l'une des revendications 1 à 5, **caractérisée en ce que** chaque demi-enveloppe comporte, sur sa face intérieure, c'est-à-dire sur sa face tournée vers l'autre demi-enveloppe, des moyens favorisant l'accrochage du coussin de compression.

7. Prothèse selon la revendication 6, **caractérisée en ce que** chaque demi-enveloppe comporte, sur sa face intérieure, deux rainures (12, 13) s'étendant sensiblement perpendiculairement l'une par rapport à l'autre et dont le point d'intersection est situé sensiblement dans la zone centrale de ladite demi-enveloppe, les deux rainures étant agencées pour permettre l'accrochage des branches du coussin de compression (7) .

8. Prothèse selon la revendication 7, **caractérisée en ce que** les deux rainures (12, 13) ménagées sur chaque demi-enveloppe présentent une forme complémentaire de celle des branches du coussin de compression (7).

9. Prothèse selon la revendication 7 ou 8, **caractérisée en ce que** les rainures (12, 13) ménagées sur chaque demi-enveloppe présentent une section en queue d'aronde.

10. Prothèse selon l'une des revendications 1 à 9, **caractérisée en ce qu'**au moins l'une des demi-enveloppes comporte, sur sa face extérieure, c'est-à-dire sa face opposée au coussin de compression (7), des pointes de fixation (15) destinées à favoriser sa fixation sur une vertèbre.

11. Prothèse selon l'une des revendications 1 à 10, **caractérisé en ce que** l'extrémité libre de chacune des première et seconde branches (9) est délimitée extérieurement par au moins une surface concave s'étendant entre les faces intérieures des première et seconde demi-enveloppes, et de préférence s'étendant de la face intérieure de la première demi-enveloppe à la face intérieure de la seconde demi-enveloppe.

12. Prothèse selon l'une des revendications 1 à 11, **caractérisé en ce qu'**au moins l'une des branches du coussin de compression (7), et de préférence au moins l'une des troisième et quatrième branches (10), comporte au moins une portion en saillie (29) s'étendant le long de la face intérieure de l'une des demi-enveloppes et dont la hauteur est inférieure à la distance séparant les deux demi-enveloppes.

13. Prothèse selon l'une des revendications 1 à 12, **caractérisé en ce que** chaque demi-enveloppe (3, 4) comporte, sur sa face intérieure, deux empreintes (32, 33) débouchant dans la face antérieure de la demi-enveloppe correspondante, les empreintes (32, 33) de la première demi-enveloppe (3) étant situées en regard des empreintes (32, 33) de la seconde demi-enveloppe (3) de manière à former deux cavités (34, 35) convergeant en direction de la face antérieure de la prothèse.

## Claims

1. An intervertebral disk prosthesis (2) including first and second rigid half-enclosures (3, 4) in the form of cups or plates, each designed to be fastened on one of the two vertebrae adjacent to the vertebra to be replaced, the first half-enclosure (3) including, in the central area thereof, a tubular piece (5) turned toward the second half-enclosure, the second half-enclosure (4) including, in the central area thereof, a lug (6) with a section smaller than that of the tubular piece, turned toward the first half-enclosure and engaged in the tubular piece, the intervertebral disk prosthesis also including a compression cushion (7) arranged between the two half-enclosures, including in the volume between the tubular piece and the lug, the compression cushion being secured to the first and second half-enclosures, **characterized in that** the compression cushion (7) includes a central portion (8) extending between the central areas of the first and second half-enclosures (3, 4) along an axis substantially perpendicular to the first and second half-enclosures, first and second branches (9) extending transversely from the central portion (8) and substantially in the medio-lateral plane of the prosthesis, and third and fourth branches (10) extending transversely from the central portion (8) and substantially in the anteroposterior plane of the prosthesis, and **in that** each half-enclosure (3, 4) has, on the surface thereof turned toward the other half-enclosure and near the free end of each of the first and second branches (9) of the compression cushion, at least one gripping finger (26, 27a, 27b), the gripping fingers situated near a same branch of the compression cushion (7) being angularly offset in relation to the central portion (8) of the compression cushion and delimiting a space (28) with said branch, the space being designed for the passage of a gripping arm of an ancillary.

2. The prosthesis according to claim 1, **characterized in that** the first, second, third and fourth branches (9, 10) of the compression cushion (7) extend substantially perpendicular to the axis of the central portion (8).

3. The prosthesis according to claim 1 or 2, **characterized in that** the length of the third and fourth branches (10) is smaller than that of the first and second branches (9), and preferably about 20% smaller than that of the first and second branches (9).

4. The prosthesis according to one of claims 1 to 3, **characterized in that** the width of each branch (9, 10) decreases continuously toward the free end of said branch.

5. The prosthesis according to one of claims 1 to 4, **characterized in that** the compression cushion is made in a single piece.

6. The prosthesis according to one of claims 1 to 5, **characterized in that** each half-enclosure has, on the inner surface thereof, i.e. the surface thereof turned toward the other half-enclosure, means favoring catching of the compression cushion.

7. The prosthesis according to claim 6, **characterized in that** each half-enclosure has, on the inner surface thereof, two grooves (12, 13) extending substantially perpendicular to one another and whereof the point of intersection is situated substantially in the central area of said half-enclosure, the two grooves being arranged to allow catching of the branches of the compression cushion (7).

8. The prosthesis according to claim 7, **characterized in that** the two grooves (12, 13) formed on each half-enclosure have a shape complementary to that of the branches of the compression cushion (7).

9. The prosthesis according to claim 7 or 8, **characterized in that** the grooves (12, 13) formed on each half-enclosure have a dovetail section.

10. The prosthesis according to one of claims 1 to 9, **characterized in that** at least one of the half-enclosures has, on the outer surface thereof, i.e. the surface thereof opposite the compression cushion (7), fastening points (15) designed to favor its fastening on a vertebra.

11. The prosthesis according to one of claims 1 to 10, **characterized in that** the free end of each of the first and second branches (9) is outwardly delimited by at least one concave surface extending between the inner surfaces of the first and second half-enclosures, and preferably extending from the inner surface of the first half-enclosure to the inner surface of the second half-enclosure.

12. The prosthesis according to one of claims 1 to 11, **characterized in that** at least one of the branches of the compression cushion (7), and preferably at least one of the third and fourth branches (10), has at least one protruding portion (29) extending along the inner surface of one of the half-enclosures and the height of which is smaller than the distance separating the two half-enclosures.

13. The prosthesis according to one of claims 1 to 12, **characterized in that** each half-enclosure (3, 4) has, on the inner surface thereof, two cavities (32, 33) emerging in the front surface of the corresponding half-enclosure, the cavities (32, 33) of the first half-enclosure (3) being situated across from the cavities (32, 33) of the second half-enclosure (3) so as to form two cavities (34, 35) converging toward the front surface of the prosthesis.

## Patentansprüche

1. Bandscheibenprothese (2), die erste und zweite starre Halbhüllen (3, 4) in Form von Schalen oder Platten aufweist, die jeweils dazu bestimmt sind, auf einem der zwei Wirbel in Nachbarschaft der zu ersetzenden Bandscheibe befestigt zu sein, wobei die erste Halbhülle (3) in ihrer zentralen Zone ein rohrförmiges Teil (5) aufweist, das in Richtung der zweiten Halbhülle gedreht ist, wobei die zweite Halbhülle (4) in ihrer zentralen Zone einen Zapfen (6) mit einem kleiner Querschnitt als das rohrförmige Teil aufweist, der in Richtung der ersten Halbhülle gedreht ist und in das rohrförmige Teil eingreift, wobei die Bandscheibenprothese ferner ein Kompressionskissen (7) aufweist, das zwischen den zwei Halbhüllen angeordnet ist, inklusive in dem Volumen, das zwischen dem rohrförmigen Teil und dem Zapfen inbegriffen ist, wobei das Kompressionskissen mit der ersten und zweiten Halbhülle verbunden ist, **dadurch gekennzeichnet, dass** das Kompressionskissen (7) einen zentraler Abschnitt (8) aufweist, der sich zwischen den zentralen Zonen der ersten und zweiten Halbhülle (3, 4) gemäß einer Achse etwa senkrecht zur ersten und zweiten Halbhülle erstreckt, wobei sich ein erster und ein zweiter Arm (9) quer ab dem zentralen Abschnitt (8) und etwa in der mediolateralen Ebene der Prothese erstrecken, und wobei sich ein dritter und vierter Arm (10) quer ab dem zentralen Abschnitt (8) und etwa in der anteroposterioren Ebene der Prothese erstrecken, und dass jede Halbhülle (3, 4) auf ihrer Seite, die zur anderen Halbhülle gedreht ist und in der Nähe des freien Endes jedes ersten und zweiten Arms (9) des Kompressionskissens mindestens einen Greiffinger (26, 27a, 27b) aufweist, wobei die Greiffinger, die sich in der Nähe desselben Arms des Kompressionskissens (7) befinden, im Verhältnis zum zentralen Abschnitt (8) des Kompressionskissens winklig versetzt sind und mit dem Arm einen Raum (28) begrenzen, der für den Durchgang eines Greifarms eines Instruments bestimmt ist.

2. Prothese nach Anspruch 1, **dadurch gekennzeichnet, dass** sich der erste, zweite, dritte und vierte Arm (9, 10) des Kompressionskissens (7) etwa senkrecht zur Achse des zentralen Abschnitts (8) erstrecken.

3. Prothese nach Anspruch 1 oder 2, **dadurch gekennzeichnet, dass** die Länge des dritten und vierten Arms (10) kleiner ist als die Länge des ersten und zweiten Arms (9) und vorzugsweise um zirka 20 % geringer ist als die des ersten und zweiten Arms (9).

4. Prothese nach einem der Ansprüche 1 bis 3, **dadurch gekennzeichnet, dass** die Breite jedes Arms (9, 10) in Richtung des freien Endes des Arms kontinuierlich abnimmt.

5. Prothese nach einem der Ansprüche 1 bis 4, **dadurch gekennzeichnet, dass** das Kompressionskissen aus einem einzigen Stück gefertigt ist.

6. Prothese nach einem der Ansprüche 1 bis 5, **dadurch gekennzeichnet, dass** jede Halbhülle auf ihrer Innenseite, das heißt auf ihrer zur anderen Halbhülle gedrehten Seite, Mittel aufweist, die das Festhaken des Kompressionskissens fördern.

7. Prothese nach Anspruch 6, **dadurch gekennzeichnet, dass** jede Halbhülle auf ihrer Innenseite zwei Rillen (12, 13) aufweist, die sich etwa im rechten Winkel im Verhältnis zueinander erstrecken und deren Schnittpunkt sich etwa in der zentralen Zone der Halbhülle befindet, wobei die zwei Rillen ausgebildet sind, um das Festhaken der Arme des Kompressionskissens (7) zu erlauben.

8. Prothese nach Anspruch 7, **dadurch gekennzeichnet, dass** die zwei Rillen (12, 13), die in jede Halbhülle eingearbeitet sind, eine komplementäre Form zu der Form der Arme des Kompressionskissens (7) aufweisen.

9. Prothese nach Anspruch 7 oder 8, **dadurch gekennzeichnet, dass** die Rillen (12, 13), die in jede Halbhülle eingearbeitet sind, einen schwalbenschwanzförmigen Querschnitt aufweisen.

10. Prothese nach einem der Ansprüche 1 bis 9, **dadurch gekennzeichnet, dass** mindestens eine der Halbhüllen auf ihrer Außenseite, das heißt auf ihrer dem Kompressionskissen (7) entgegengesetzten Seite, Fixierspitzen (15) aufweist, die dazu bestimmt sind, ihre Fixierung auf einem Wirbel zu unterstützen.

11. Prothese nach einem der Ansprüche 1 bis 10, **dadurch gekennzeichnet, dass** das freie Ende jedes der ersten und zweiten Arme (9) außen von mindestens einer konkaven Fläche begrenzt wird, die sich zwischen den Innenseiten der ersten und zweiten Halbhülle erstreckt, wobei sie sich vorzugsweise von der Innenseite der ersten Halbhülle zur Innenseite der zweiten Halbhülle erstreckt.

12. Prothese nach Anspruch einem der Ansprüche 1 bis 11, **dadurch gekennzeichnet, dass** mindestens einer der Arme des Kompressionskissens (7) und vorzugsweise mindestens einer des dritten und vierten Arms (10) mindestens einen hervorstehenden Abschnitt (29) aufweist, der sich entlang der Innenseite einer der Halbhüllen erstreckt und dessen Höhe kleiner ist als der Abstand, der die zwei Halbhüllen trennt.

13. Prothese nach einem der Ansprüche 1 bis 12, **dadurch gekennzeichnet, dass** jede Halbhülle (3, 4) auf ihrer Innenseite zwei Eindrückungen (32, 33) aufweist, die in die Vorderseite der entsprechenden Halbhülle münden, wobei sich die Eindrückungen (32, 33) der ersten Halbhülle (3) gegenüber Eindrückungen (32, 33) der zweiten Halbhülle (3) derart befinden, dass zwei in Richtung der Vorderseite der Prothese konvergierende Hohlräume (34, 35) gebildet werden.
